# EUROPEAN PATENT APPLICATION

(11) **EP 1 375 659 A1**
(43) Date of publication of application: **02.01.2004**
(21) Application number: 02405500.6
(22) Date of filing: 18.06.2002
(51) Int. Cl.: C12N 15/10

(54) **Soluble capsules containing stock mixtures for easy preparation of chemical or biological solutions**

(71) Applicant: Laboratorio di Diagnostica Moleculare, 6932 Breganzona (CH)
(72) Inventor: Balmelli, Tiziano, 6992 Cimo (CH); Soldati, Gianni, 6968 Sonvico (CH)
(74) Representative: Liebetanz, Michael, Dipl.-Phys.

(57) **Abstract**

A storage system for chemical or biological stock solutions in form of encapsulated chemical or biological stock mixtures, which can be readily used in the preparation of any chemical or biological solution of choice, e.g. buffers, bacterial media, gel solutions and other commonly used stock solutions, that are typically employed in analytical and diagnostic techniques in the life sciences.

## Description

The present invention provides a storage system for chemical or biological stock solutions in form of encapsulated chemical or biological stock mixtures, which can be readily used in the preparation of any chemical or biological solution of choice, e.g. buffers, bacterial media, gel solutions and other commonly used stock solutions, that are typically employed in analytical and diagnostic techniques in the life sciences.

The preparation of any chemical or biological solution, e.g. buffers, bacterial media, gel solutions and the like, to be used in analytical and diagnostic techniques is a daily requirement in every basic research and diagnostic laboratory in the life sciences, such as in the areas of molecular biology and cell biology. These solutions are used in techniques that may range from simple procedures, e.g. the preparation of cell culture media, to highly sophisticated techniques, e.g. PCR, and their successful performance depends on an accurate and reliable preparation of the chemical or biological solutions employed.

Current preparation of any of these solutions involves time-consuming and laborious manipulation of the components that make up the chemical or biological solution of choice. At the same time it requires safe and cautious manipulation in case of hazardous materials, as well as clean and accurate handling to avoid the occurrence of experimental errors or potential contamination, which may impair the outcome of the experiments and reliability of the results.

Applicants have now found that these disadvantages can be overcome by using the encapsulated chemical or biological stock mixtures of the present invention which can readily be used in the preparation of any chemical or biological solution of choice, that are typically employed in analytical and diagnostic techniques in the life sciences. The use of these encapsulated chemical or biological stock mixtures leads to a simplification of such techniques through their instantaneous use, the ease and safe handling of the materials utilised and the prevention of contamination.

Thus in a first aspect the present invention provides a storage system for chemical or biological solutions in form of encapsulated chemical or biological stock mixtures, which allows rapid preparation of virtually any type of buffer solution to be employed in virtually any type of analytical and diagnostic technique. Moreover, such encapsulated chemical or biological stock mixtures may be easily prepared, are user-friendly and cost-effective and thus may improve the efficiency of any analytical and diagnostic technique it is used for.

The chemical or biological solutions contemplated include any chemical or biological solution typically used in the life sciences, such as buffers or buffer mixtures, media, e.g. bacterial media, gel solutions, any other commonly used stock solutions, as well as combinations thereof.

Examples of buffers include commonly used buffers well known in the art (see for example Sambrook, Fritsch, Maniatis, Molecular Cloning, A Laboratory manual, 2^{nd} Edition, Vol 3, Appendix B), such as various buffers used in molecular biology techniques, and include for example
(a) gel loading buffers, e.g. denaturing and non-denaturing, e.g. SDS loading buffers,
(b) running buffers,
(c) sequencing buffers,
(d) elution buffers, e.g. electroelution buffers,
(e) stocking buffers,
(f) lysis buffers, e.g. alkaline lysis buffers, e.g. NaOH/SDS-buffer,
(g) wash buffers,
(h) dilution buffers,
(i) extraction buffers,
(j) ligation buffers
and the like.

Particularly preferred buffers include e.g. Tris-buffers, e.g. Tris-glycine-, Tris-NaCl-, Tris-EDTA(TE)-, Tris-borate-EDTA (TBE)-, TAE-buffer and Tris-buffered saline (TBS), HEPES-, MOPS-, PIPES-, MES-, PBS-, PBP- buffers and the like.

It is understood that any of these buffers may be present in combination with a medium, a gel solution such as agarose or any other commonly used stock solution.

It is further understood that any of the above buffer solutions may be present in form of a buffer mixture comprising in addition further components, such as for example pH modifiers, indicators (dyes), surfactants, stabilizers, sugars, proteins, and the like, depending on their intended use in a particular technique. Such components which may be present in buffers at concentrations well-known in the art, e.g. 1 x to 10 x, include, e.g.
(a) pH modifiers, e.g. sodium hydroxide, hydrochloric acid, or potassium phosphate, at a concentration of e.g. 1N to 5N, which may be added to the composition to bring the pH within about 6 to 8, preferably within 6.5 to 7.5, and most preferably about pH 7;
(b) indicators, e.g. a tracking dye, e.g. a water-soluble dye, e.g. bromophenol blue, xylene cyanole, bromocresol red or cresol red, at a concentration of e.g. 1 x to 5 x, preferably 1 x;
(c) surfactants, e.g. non-ionic surfactants, such as NP40 and Tween 20, or ionic surfactants, at a concentration of e.g. 1 x to 5 x;
(d) stabilizers, e.g. gelatine, BSA, Thesit (polyoxyethylene-9-lauryl ether), PEG-8000(polyethyleneglycol-8000) or a polyol, such as glycerol, glucose, mannitol, galacitol, glucitol and sorbitol, at a concentration of e.g. 0.5 % to 20 %;
(e) sugars, e.g. maltose;
(f) proteins, e.g. bovine serum albumin, immunoglobulins and peptides, at a concentration of e.g. 0.1 % to 10 %;
(g) nucleotides;
(h) denaturants, e.g. urea and formamide, at a concentration of e.g. 1 x to 5 x;
and the like.

The above buffers may be employed in various, typically used concentrations, e.g. 1 x to 50 x, preferably 1 x to 10 x, to yield working buffer solutions or stock solutions of desired concentration.

Examples of bacterial media include commonly used bacterial media well known in the art at well-known concentrations, e.g. 1 x to 10 x, (see for example Sambrook, Fritsch, Maniatis, Molecular Cloning, A Laboratory manual, 2^{nd} Edition, Vol 3, Appendix A), such as liquid media, e.g. LB Medium, NZCYM Medium, NZYM Medium, NZM Medium, Terrific Broth, SOB Medium, SOC Medium, 2xYT Medium, M9 Minimal Medium and Media containing agar or agarose.

Typical examples of agar include, e.g. Anaerobiose-agar, Hefeextrakt-Cystein-Blut (HCB-)agar, Mueller-Hinton-agar, Salmonella-Shigella-(SS)agar, Thayer-Martin-agar, Anaerobic Bloodagar according to CDC, Hefeextrakt-Cystein-Blut modified with Hämin (HCBH-)agar, and the like.

Examples of gel solutions include for example solutions of agarose, e.g. standard agarose, agarose high, agarose medium, agarose low melt; agar solutions, e.g. DNA agar; acrylamide/bisacrylamide, and the like, which can be encapsulated alone or in combination with suitable buffer components. Said gel solutions may be used at concentrations well-known in the art, e.g. at concentrations to yield a final concentration of a gel solution of choice ranging from e.g. 0.5 % to 5 %.

The capsules so prepared are ready to be dissolved in exact amounts of bidest water, e.g. 50 ml, 75 ml, 100 ml depending on the size of the gel.

Examples of other stock solutions include commonly used stock solutions well known in the art (see for example Sambrook, Fritsch, Maniatis, Molecular Cloning, A Laboratory manual, 2^{nd} Edition, Vol 3, Appendix B) at typically used concentrations, such as
(a) salt solutions, e.g. physiological NaCl-solution, at a concentration of e.g. 0.9 % to 10 %, ammonium acetate solution , at a concentration of e.g. 1 x to 5 x, ammonium persulfate solution, magnesium acetate solution, MgCl₂- or CaCl₂-solution;
(b) nucleotid solutions, e.g. dNTP-solution,
(c) sugar solutions, at a concentration of e.g. 0.9 % to 10 %, e.g. IPTG-, x-gal-solution
and the like.

The encapsulated chemical or biological stock mixtures of the present invention may be prepared by mixing and filling appropriate amounts of the components of the chemical or biological solution of choice into a capsule, preferably a gelatine capsule, e.g. a hard gelatine capsule, of for example 2g, 3g or 5g size.

As the need for a particular chemical or biological solution of choice arises, the preparation of a desired buffer solution from these capsules may comprise either (i) dissolving an appropriate number of capsules in their entirety in an appropriate amount of liquid, e.g. bidest water, under continuous stirring and optionally heating for a certain amount of time, e.g. 1 minute, in a microwave or (ii) opening an appropriate number of capsules and mixing their content with an appropriate amount of liquid, e.g. bidest water, under continuous stirring and optionally heating for a certain amount of time, e.g. 1 minute, in a microwave, to yield a chemical or biological solution of desired composition and concentration.

As shown above and demonstrated in the following representative examples, there are numerous advantages of the storage system for chemical or biological solutions in form of encapsulated chemical or biological stock mixtures of the present invention, such as, fast preparation of virtually any chemical or biological solution, safe handling of the materials utilised, high accuracy and thus a reduction in experimental errors and/or potential contamination. All of these advantages improve the efficiency while simplifying the procedures for preparing the chemical or biological solutions of choice. Moreover, the use of such encapsulated chemical or biological stock mixtures of the present invention is of economical value and introduces convenient packaging alternatives.

The present invention is further illustrated by the following examples, which are in no way meant to limit the scope of the invention or to exclude any other embodiments, adaptions, variations and equivalent arrangements.

### Example 1: Gelatine capsules comprising 0.5x TBE

0.2724 g of Tris base, 0.1391 g of boric acid, 0.1861 g of EDTA, and 0.02g of NaOH pellets were mixed and dispensed in 1 g size hard gelatine capsules. To obtain 100 ml of 0.5 x TBE-solution 2 capsules were dissolved in 100 ml of bidest water under continuous stirring.

### Example 2: Gelatine capsules comprising 0.5x TBE and 1 % agarose

0.2724 g of Tris base, 0.1391 g of boric acid, 0.1861 g of EDTA, 0.02g of NaOH pellets and 0.5 g agarose were mixed and dispensed in 2 g size hard gelatine capsules. To obtain 100 ml of 0.5 x TBE-solution containing 1 % agarose 2 capsules were dissolved in 100 ml of bidest water under continuous stirring.

## Claims

1. A capsule comprising a chemical or biological stock mixture for use in the preparation of a chemical or biological solution.

2. A capsule according to claim 1, wherein the chemical or biological stock mixture comprises components of a buffer, a medium, a gel solution, any other commonly used stock solution or a combination thereof.

3. A capsule according to claim 2, wherein the buffer is a gel loading buffer, a running buffer, a sequencing buffer, an elution buffer, a stocking buffer, a lysis buffer, a wash buffer, a dilution buffer, an extraction buffer or a ligation buffer.

4. A capsule according to claims 2 or 3 further comprising a pH modifier, an indicator, a surfactant, a stabilizer, a sugar, a protein, a nucleotid or a denaturant.

5. A capsule according to anyone of claims 2 to 4 further comprising a gel solution.

6. A capsule according to claim 2, wherein the medium is a bacterial medium.

7. A capsule according to claim 2, wherein the gel solution is an agarose-, agar-, acrylamide- or acrylamide/bisacrylamide solution.

8. A capsule according to claim 2, wherein the commonly used stock solution is a salt solution, a nucleotid solution or a sugar solution.

9. A capsule according to any preceding claim wherein the capsule is a gelatine capsule.

10. Use of a capsule according to any preceding claim in the preparation of a chemical or biological solution.
